# EUROPEAN PATENT APPLICATION

(11) **EP 3 263 142 A1**
(43) Date of publication of application: **03.01.2018**
(21) Application number: 16755630.7
(22) Date of filing: 25.02.2016
(51) Int. Cl.: A61L 27/00, A61L 15/64, A61P 17/00, A61P 41/00

(54) **MEDICAL/COSMETIC MATERIAL AND ADHESION PREVENTING MATERIAL**

(30) Priority: 27.02.2015 JP 2015038009
(71) Applicant: Dainichiseika Color & Chemicals Mfg. Co., Ltd., Chuo-ku Tokyo 103-8383 (JP)
(72) Inventor: ISONO Yasuyuki, Tokyo 103-8383 (JP); NOISHIKI Yasuharu, Yokohama-shi Kanagawa 236-0005 (JP)
(74) Representative: Blodig, Wolfgang
(86) International application number: PCT/JP2016/055656
(87) International publication number: WO 2016/136886

(57) **Abstract**

There is provided a medical/cosmetic material that retains the properties which are inherent in a polyanionic polysaccharide being a raw material, that has a high level of safety because there is no need to use a chemical crosslinking agent during production, and that makes it possible to place a powder of a water-insoluble polyanionic polysaccharide at an affected area uniformly. The medical/cosmetic material includes a base and a powder dispersed in the base, in which the base contains a water-soluble salt of a first polyanionic polysaccharide, and the powder is formed by water-insolubilizing a powder of a water-soluble salt of a second polyanionic polysaccharide with a treatment liquid containing an acid anhydride.

## Description

### Technical Field

The present invention relates to a medical/cosmetic material and an anti-adhesion material.

### Background Art

It is known that polyanionic polysaccharides such as hyaluronic acid and alginic acid exhibit moderate viscosity, adhesiveness, moisture retention, and biocompatibility. Therefore, these polyanionic polysaccharides and salts thereof are widely used as a raw material for medical materials, food materials, cosmetic materials, and the like.

Hyaluronic acid among others is excellent in its characteristic physical properties such as water retention and has a high level of safety and biocompatibility, so that it is used in various applications such as foods, cosmetics, and pharmaceutical products. For example, in the medical field, hyaluronic acid is used in raw materials for joint lubricants and anti-adhesion materials. However, sodium hyaluronate to be a raw material has a high water-solubility and therefore needs to be subjected to certain insolubilization treatment depending on the application.

Various studies on the method for water-insolubilizing sodium hyaluronate through crosslinking reaction making use of a carboxy group have been made so far. For example, Patent Literature 1 describes a method for producing a water-insoluble derivative of a polyanionic polysaccharide such as hyaluronic acid and carboxymethyl cellulose through crosslinking reaction using a carbodiimide.

In addition, Patent Literatures 2 and 3 describe a method for water-insolubilizing a polyanionic polysaccharide such as hyaluronic acid and carboxyalkyl cellulose by forming ionic bonds using a polyvalent cation. Further, Patent Literature 4 describes a method for obtaining a water-insolubilized film by subjecting carboxymethyl cellulose to ion exchange using a metal salt.

Patent Literature 5 describes a method for water-insolubilizing sodium hyaluronate by cooling a sodium hyaluronate aqueous solution to -20°C under an acidic condition to form intramolecular crosslinks. In addition, Patent Literature 6 describes acetylation through reaction of hyaluronic acid in a powder form with acetic anhydride in the presence of concentrated sulfuric acid. Further, Patent Literature 7 describes a method for producing hyaluronic acid gel using an acidic liquid containing an alcohol.

### Citation List

### Patent Literature

Patent Literature 1: National Publication of International Patent Application No. 2003-518167
Patent Literature 2: Japanese Patent Application Laid-Open No. 5-124968
Patent Literature 3: Japanese Patent Application Laid-Open No. 2008-13510
Patent Literature 4: Japanese Patent Application Laid-Open No. 6-128395
Patent Literature 5: Japanese Patent Application Laid-Open No. 2003-252905
Patent Literature 6: Japanese Patent Application Laid-Open No. 8-53501
Patent Literature 7: Japanese Patent Application Laid-Open No. 5-58881

### Summary of Invention

### Technical Problem

However, a crosslinking agent is used in the method described in Patent Literature 1, and therefore it is often difficult to apply the method for applications where the safety should be taken into consideration because products are applied to human bodies, such as pharmaceutical products. In addition, the extent of water-insolubility for the obtained films and the like are not described at all in Patent Literatures 2 to 4.

Further, in the method described in Patent Literature 5, the pH of the sodium hyaluronate aqueous solution needs to be adjusted at about 1.2, and the viscosity increases outstandingly, so that handling at the time of shaping and the like is difficult. In addition, freeze drying is conducted for long hours, so that there has also been a problem in terms of cost of electric power required for cooling. Further, when the sodium hyaluronate aqueous solution is stored under an acidic condition, the viscosity increases rapidly to make the shaping difficult, so that the application may be limited. It is to be noted that in Patent Literature 5, the intramolecular crosslinked structure is identified, but the extent of insolubilization is not referred to.

In addition, the extent of water-insolubility for the obtained acetylated product of hyaluronic acid is not described at all in Patent Literature 6. Further, the hyaluronic acid gel obtained by the method described in Patent Literature 7 contains a large amount of water and therefore is hard even to lift. Thus, it is difficult to insolubilize the shaped body while keeping the shape of the shaped body.

The present invention has been completed in consideration of the problems of the conventional techniques, and the subject matter of the present invention is to provide a medical/cosmetic material: that retains the properties which are inherent in a polyanionic polysaccharide being a raw material; that has a high level of safety because there is no need to use a chemical crosslinking agent during production; and that makes it possible to place a powder of a water-insoluble polyanionic polysaccharide at an affected area uniformly.

### Solution to Problem

That is, according to the present invention, a medical/cosmetic material described below is provided.
[1] A medical/cosmetic material including:
   a base containing a water-soluble salt of a first polyanionic polysaccharide; and
   a powder dispersed in the base and formed by water-insolubilizing a powder of a water-soluble salt of a second polyanionic polysaccharide with a treatment liquid containing an acid anhydride.
[2] The medical/cosmetic material according to [1], wherein the base is an aqueous solution of the water-soluble salt of the first polyanionic polysaccharide.
[3] The medical/cosmetic material according to [1], wherein the base is a film-like shaped body made of a raw material containing the water-soluble salt of the first polyanionic polysaccharide.
[4] The medical/cosmetic material according to any one of [1] to [3], wherein the first polyanionic polysaccharide and the second polyanionic polysaccharide are each at least one selected from the group consisting of hyaluronic acid, carboxymethyl cellulose, and alginic acid.
[5] The medical/cosmetic material according to any one of [1] to [4], wherein the acid anhydride is at least any one of acetic anhydride and propionic anhydride.
   In addition, according to the present invention, an anti-adhesion material described below is provided.
[6] An anti-adhesion material containing a polyhydric alcohol or a polyhydric alcohol aqueous solution retained in the medical/cosmetic material according to any one of [3] to [5].

### Advantageous Effects of Invention

The medical/cosmetic material according to the present invention retains the properties which are inherent in a polyanionic polysaccharide being a raw material, has a high level of safety because there is no need to use a chemical crosslinking agent during production, and makes it possible to place a powder of a water-insoluble polyanionic polysaccharide at an affected area uniformly.

### Brief Description of Drawing

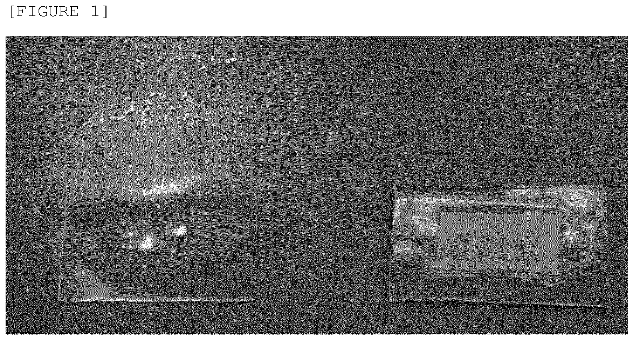

[Figure 1] Figure 1 shows a photograph that shows the state of a powder of sodium hyaluronate (left-hand side) and the state of a hyaluronic acid powder-sodium hyaluronate composite film obtained in Example 2 (right-hand side).

### Description of Embodiments

Hereinafter, embodiments according to the present invention will be described; however, the present invention is not limited to the embodiments below.

### (Medical/Cosmetic Material)

A medical/cosmetic material according to the present invention includes: a base containing a water-soluble salt of a first polyanionic polysaccharide; and a powder dispersed in the base. The first polyanionic polysaccharide is a polysaccharide having one or more negatively charged anionic groups such as a carboxy group and a sulfonic acid group in the molecular structure thereof. In addition, the water-soluble salt of the first polyanionic polysaccharide is a salt formed from at least a part of the anionic groups in the first polyanionic polysaccharide. It is to be noted that the anionic group in the first polyanionic polysaccharide may be the one that is introduced in a molecule of a polysaccharide.

Specific examples of the first polyanionic polysaccharide include carboxyalkyl cellulose such as carboxymethyl cellulose and carboxyethyl cellulose, carboxymethyl starch, carboxymethylamylose, chondroitin sulfate (including chondroitin-4-sulfate and chondroitin-6-sulfate), hyaluronic acid, heparin, heparin sulfate, heparan sulfate, alginic acid, pectin, carrageenan, dermatan sulfate, and dermatan-6-sulfate. These first polyanionic polysaccharides can be used singly or in a combination of two or more.

Examples of the water-soluble salt of the first polyanionic polysaccharide include inorganic salts, ammonium salts, and organic amine salts. Specific examples of the inorganic salts include: alkali metal salts such as sodium salts and potassium salts; alkali earth metal salts such as calcium salts; and metal salts such as zinc salts and iron salts.

Specific examples of the base include (i) an aqueous solution of a water-soluble salt of a first polyanionic polysaccharide and (ii) a film-like shaped body made of a raw material containing a water-soluble salt of a first polyanionic polysaccharide. In the case where the base is the aqueous solution (i), the medical/cosmetic material according to the present invention is in a liquid form, a paste form, or the like. In addition, in the case where the base is the film-like shaped body (ii), the medical/cosmetic material according to the present invention is in a solid form (film form, sheet form), or the like.

The powder dispersed in the base is formed by water-insolubilizing a powder of a water-soluble salt of a second polyanionic polysaccharide with a treatment liquid containing an acid anhydride. As the second polyanionic polysaccharide, the same polyanionic polysaccharides as the first polyanionic polysaccharides can be used. The first polyanionic polysaccharide and the second polyanionic polysaccharide may be the same or different.

The treatment liquid which is used for water-insolubilizing the powder of the water-soluble salt of the second polyanionic polysaccharide contains an acid anhydride. Specific examples of the acid anhydride include acetic anhydride, propionic anhydride, succinic anhydride, butyric anhydride, phthalic anhydride, and maleic anhydride. Among them, acetic anhydride and propionic anhydride are preferable. These acid anhydrides can be used singly or in a combination of two or more.

It is preferable that the treatment liquid further contain at least one medium of water and a water-soluble organic solvent, and it is also preferable that the acid anhydride be dissolved or dispersed in the medium. By using the treatment liquid in which the acid anhydride is dissolved or dispersed in the medium, the powder of the water-soluble salt of the second polyanionic polysaccharide can be water-insolubilized sufficiently and immediately.

Specific examples of the water-soluble organic solvent include methanol, ethanol, propanol, dimethyl sulfoxide (DMSO), acetonitrile, and tetrahydrofuran. Among them, methanol, ethanol, and dimethyl sulfoxide are preferable. These water-soluble organic solvents can be used singly or in a combination of two or more.

The concentration of the acid anhydride in the treatment liquid is usually 0.1 to 50% by mass and is preferably 5 to 30% by mass. When the concentration of the acid anhydride is less than 0.1% by mass, the extent of water-insolubilization is insufficient, or there is a tendency that the water-insolubilization takes a long time. On the other hand, when the concentration of the acid anhydride exceeds 50% by mass, there is a tendency that the effects hit the ceiling.

It is preferable that the treatment liquid contain water as a medium from the viewpoint of water-insolubilizing the powder of the water-soluble salt of the second polyanionic polysaccharide further sufficiently and immediately. The content of water in the treatment liquid is preferably 0.01 to 50% by mass, more preferably 5 to 20% by mass. When the content of water in the treatment liquid is less than 0.01% by mass, the water-insolubilization may be insufficient for the solvents other than methanol. In addition, when the content of water in the treatment liquid exceeds 50% by mass, the powder of the water-soluble salt of the second polyanionic polysaccharide may dissolve easily.

The temperature during the water-insolubilization treatment is not particularly limited as long as the temperature does not exceed the boiling point of the treatment liquid. It is preferable to set the temperature during the water-insolubilization treatment at 0 to 80°C, more preferably 0 to 70°C, and particularly preferably room temperature (25°C) to 60°C from the viewpoint of suppressing the decomposition and denaturation of the polyanionic polysaccharide and from the viewpoint of suppressing the volatilization of the medium, byproducts, and the like. However, when the treatment is conducted under the condition in which the treatment liquid does not volatilize during the water-insolubilization treatment, for example, the treatment is conducted with a heat press or a heat roller, the medical material can be obtained in a shorter time without undergoing the decomposition and denaturation, and the like. For example, in the case where the water-insolubilization treatment is conducted with a heat press or a heat roller, it is preferable that the temperature during the water-insolubilization treatment be set at 50 to 90°C, and the treatment time be set for 30 minutes or shorter. After the water-insolubilization treatment is completed, the powder to be dispersed in the base can be obtained, if necessary, through washing or the like with water or a water-soluble organic solvent.

The reaction that is supposed to progress when the powder of a sodium salt of the polyanionic polysaccharide is treated with an alcoholic solution of acetic anhydride is shown below. It is to be noted that the supposed reaction can be one of the factors for water-insolubilization, but there is a possibility that the water-insolubilization is achieved by a combination with another factor for water-insolubilization or by a totally different factor. That is, the present invention is not limited at all by the supposed reaction described below.

R1-COONa + (CH₃CO)₂O + R₂-OH → R1-COOH + CH₃COONa + CH₃COOR₂ ^{▪▪▪} (1)

In the reaction formula (1), R₁ represents the main chain of the polyanionic polysaccharide, and R₂ represents the main chain of the alcohol. Acetic anhydride, when cleaved in the presence of an alcohol, deprives sodium of the polyanionic polysaccharide and the carboxy groups change into an acid form from a sodium salt form. The change can be confirmed by measuring the Na content or by titration with an alkaline solution.

In the case where water exists in the reaction system, it is envisaged that the reaction represented by the following reaction formula (2) progresses in addition to and in parallel with the reaction represented by the reaction formula (1) and the carboxy groups change into an acid form from a sodium salt form.

R₁-COONa + (CH₃CO)₂O + H₂O → R₁-COOH + CH₃COONa + CH₃COOH ^{▪▪▪} (2)

It is to be noted that all the anionic groups in a molecule may not necessarily be in the acid form in the obtained medical/cosmetic material.

It is extremely difficult to obtain a powder that is sufficiently water-insolubilized even when the powder of the water-soluble salt of the polyanionic polysaccharide is immersed in an inorganic acid such as hydrochloric acid or an organic acid such as acetic acid. In addition, a water-insolubilized powder cannot be obtained even when the acid anhydride in the treatment liquid is replaced with an acid that corresponds to the acid anhydride. From these facts, it is envisaged that the water-insolubilization is achieved by a different factor combined with the factor that the anionic groups in the polyanionic polysaccharide change into the acid form.

The medical/cosmetic material in a liquid form or a paste form can be obtained by mixing: the powder obtained by water-insolubilizing the powder of the water-soluble salt of the second polyanionic polysaccharide; and the aqueous solution of the water-soluble salt of the first polyanionic polysaccharide, and appropriately stirring the resultant mixture. In addition, the medical/cosmetic material in a solid form (film form, sheet form) can be obtained by mixing: the powder obtained by water-insolubilizing the powder of the water-soluble salt of the second polyanionic polysaccharide; and the raw material containing the water-soluble salt of the first polyanionic polysaccharide, and then shaping the resultant mixture in a film form.

The medical/cosmetic material in a liquid form or a paste form can be used, for example, as a joint function improving agent or a cosmetic material for subcutaneous infusion. When the medical/cosmetic material in a liquid form or a paste form according to the present invention is injected into or applied on an affected area, the base is decomposed and absorbed in a living body, but the powder of the water-insoluble polyanionic polysaccharide is left as it is in the affected area. Therefore, by using the medical/cosmetic material in a liquid form or a paste form according to the present invention, the powder of the water-insoluble polyanionic polysaccharide can be placed more uniformly and in a more suitable range than in the case where the powder of the water-insoluble polyanionic polysaccharide is injected into or applied on the affected area by a spraying method or the like. In addition, the powder of the water-insoluble polyanionic polysaccharide is gradually changed into a water-soluble salt due to the neutralization action in the body to be absorbed and metabolized. Therefore, by using the medical/cosmetic material in a liquid form or a paste form according to the present invention, an extended release system in which the effects of the medical/cosmetic material are exhibited and maintained slowly at a treatment area (affected area) over a long period of time is achieved.

In addition, the medical/cosmetic material in a solid form (film form, sheet form) can be used, for example, as a raw material for anti-adhesion materials. When the anti-adhesion material obtained using the medical/cosmetic material in a solid form (film form, sheet form) according to the present invention is arranged at an affected area, the base is decomposed and absorbed in a body, but the powder of the water-insoluble polyanionic polysaccharide is left as it is in the affected area. Therefore, by using the medical/cosmetic material in a solid form (film form, sheet form) according to the present invention, the powder of the water-insoluble polyanionic polysaccharide can be placed more uniformly and in a more suitable range than in the case where the powder of the water-insoluble polyanionic polysaccharide is arranged at the affected area by a spraying method or the like.

There is no need to use a chemical crosslinking agent during the production of the medical/cosmetic material according to the present invention, and therefore a structure of a functional group or the like which is derived from the chemical crosslinking agent is not incorporated into the molecule. Therefore, the medical/cosmetic material according to the present invention retains the properties which are inherent in the polyanionic polysaccharide being a raw material and has a high level of safety. Accordingly, the medical/cosmetic material according to the present invention is suitable as an anti-adhesion material and the like. It is to be noted that in the case where the medical/cosmetic material according to the present invention is used as a constituent material for an anti-adhesion material, the thickness of the medical/cosmetic material is not particularly limited, but is preferably 20 to 200 µm, more preferably 60 to 120 µm.

The molecules of the polyanionic polysaccharide, which constitutes the medical/cosmetic material according to the present invention, are not substantially crosslinked. Further, a new covalent bond is not substantially formed in the polyanionic polysaccharide. However, it is inferred that physical bonds such as hydrogen bonds, hydrophilic bonds, and van der Walls force are formed between the molecules of the polyanionic polysaccharide. Formation of such physical bonds between the molecules of the polyanionic polysaccharide can be confirmed by measuring an infrared absorption spectrum.

The powder of the water-insoluble polyanionic polysaccharide which constitutes the medical/cosmetic material according to the present invention is stably water-insoluble in a wide pH range from acidity to alkalinity. However, the powder of the water-insoluble polyanionic polysaccharide, when brought into contact with or immersed in an aqueous medium having a pH of 12 or higher, can be dissolved easily due to dissociation of the physical bonds between the molecules.

### (Anti-Adhesion Material)

The anti-adhesion material according to the present invention contains a polyhydric alcohol or a polyhydric alcohol aqueous solution retained in the medical material/cosmetic material (provided that the base is a film-like shaped body made of a raw material containing the water-soluble salt of the first polyanionic polysaccharide). Specific examples of the polyhydric alcohol include ethylene glycol, diethylene glycol, polyethylene glycol, methyl glycerol, polyoxyethylene glycoside, maltitol, mannitol, xylitol, sorbitol, reduced sugar syrup, dipropylene glycol, butylene glycol, valine, propylene glycol, glycerin (glycerol), polyglycerin, and fatty acid esters of glycerin. Among them, polyhydric alcohols which are used in the medical field and the food field, such as glycerin, xylitol, sorbitol, and low-molecular-weight polyethylene glycols, are used suitably. With respect to these suitably usable polyhydric alcohols, products sold on the market can be used as they are. With respect to glycerin, sorbitol, and the like, it is desirable to use products which conform to the Japanese pharmacopoeia. Glycerin is a material having a high level of safety to such an extent that can also be used as injections into a vein and therefore is greatly preferable.

Examples of the method for allowing the polyhydric alcohol or the polyhydric alcohol aqueous solution to be retained in the medical/cosmetic material include a method in which the medical/cosmetic material is immersed in the polyhydric alcohol or the polyhydric alcohol aqueous solution having a predetermined concentration. That is, by immersing the medical/cosmetic material in the polyhydric alcohol aqueous solution to replace the inside of the base with the polyhydric alcohol aqueous solution, the polyhydric alcohol aqueous solution in a desired concentration is retained, so that a desired anti-adhesion material according to the present invention can be obtained. It is to be noted that the thickness of the anti-adhesion material according to the present invention is not particularly limited, but is preferably 20 to 200 µm, more preferably 60 to 120 µm.

### Examples

Hereinafter, the present invention will be described specifically based on Examples; however, the present invention is not limited to the Examples. It is to be noted that the "part (s) " and "%" in Examples and Comparative Examples are on a mass basis unless otherwise noted.

### (Example 1)

In 100 mL of an 80% ethanol aqueous solution, 1.0 g of a powder of sodium hyaluronate (molecular weight of 800000 Da) was dispersed. The resultant dispersion liquid was heated to 50°C under stirring, and 20 mL of acetic anhydride were then added thereto to stir the resultant mixture for further 1 hour. A precipitate collected by centrifugal separation was washed with ethanol and water, and was then dried and pulverized to obtain a hyaluronic acid powder. The obtained hyaluronic acid powder in an amount of 0.5 g was put into 50 mL of a 4% sodium hyaluronate (molecular weight of 800000 Da) aqueous solution. The hyaluronic acid powder was uniformly dispersed using a homogenizer to obtain a composition (medical/cosmetic material) in a paste form.

### (Example 2)

In 100 mL of an 80% ethanol aqueous solution, 1.0 g of a powder of sodium hyaluronate (molecular weight of 800000 Da) was dispersed. The resultant dispersion liquid was heated to 50°C under stirring, and 20 mL of acetic anhydride were then added thereto to stir the resultant mixture for further 1 hour. A precipitate collected by centrifugal separation was washed with ethanol and water, and was then dried and pulverized to obtain a hyaluronic acid powder. The obtained hyaluronic acid powder in an amount of 0. 5 g was dispersed in 50 mL of a 1% sodium hyaluronate (molecular weight of 800000 Da) aqueous solution, and the resultant dispersion liquid was poured into a stainless-steel tray of 12-cm length × 10-cm width and was then dried in a thermostatic chamber at 20°C to obtain a hyaluronic acid powder-sodium hyaluronate composite film (medical/cosmetic material) having a thickness of about 55 µm.

### (Evaluation)

A sheet of the hyaluronic acid powder-sodium hyaluronate composite film obtained in Example 2 was put on a gelatin sheet. When water was poured over the composite film, the film section made of sodium hyaluronate dissolved, and the water-insolubilized hyaluronic acid powder was left on the gelatin sheet uniformly. In contrast, 0.5 g of the powder of sodium hyaluronate used in Example 2 and 0.5 g of the water-insolubilized hyaluronic acid powder obtained in Example 2 were sprayed and applied on the gelatin sheet using a powder sprayer to find that both the powders were applied in a wide range and nonuniformly. Figure 1 shows a photograph that shows the state of the powder of hyaluronic acid (left-hand side) and the state of the hyaluronic acid powder-sodium hyaluronate composite film obtained in Example 2 (right-hand side).

### (Example 3)

In 100 mL of an 80% ethanol aqueous solution, 1.0 g of a powder of sodium hyaluronate (molecular weight of 800000 Da) was dispersed. The resultant dispersion liquid was heated to 50°C under stirring, and 20 mL of acetic anhydride were then added thereto to stir the resultant mixture for further 1 hour. A precipitate collected by centrifugal separation was washed with ethanol and water, and was then dried and pulverized to obtain a hyaluronic acid powder. The obtained hyaluronic acid powder in an amount of 0. 5 g was dispersed in 50 mL of a 1% sodium hyaluronate (molecular weight of 800000 Da) aqueous solution in which 0.5% of glycerin (Japanese pharmacopoeia) were blended, and the resultant dispersion liquid was poured into a stainless-steel tray of 12-cm length × 10-cm width and was then dried in a thermostatic chamber at 20°C to obtain a hyaluronic acid powder-sodium hyaluronate composite film containing glycerin. The obtained composite film was sealed in a sterilization bag and was sterilized together with the sterilization bag with an ethylene oxide gas to obtain an anti-adhesion film having a thickness of about 55 µm.

A thoracotomy was performed to a mature dog (beagle dog, female, 1.5 years old, weight of about 10 kg) after general anesthesia. The lung was exposed to the air outside for 10 minutes, and the obtained anti-adhesion film was placed on the lung directly under the wound at open chest portion, and the chest was then closed. Four weeks later, a thoracotomy was performed to the same dog after general anesthesia to find that adhesion had not occurred. In addition, the anti-adhesion film placed (implanted) in the body of the dog was found to have disappeared four weeks after the implantation. As the reason, it is inferred that the carboxy groups in hyaluronic acid, which constitutes the anti-adhesion film, were gradually neutralized by sodium ions or the like in the living body to cause the hyaluronic acid to change into a water-soluble hyaluronic acid salt, so that the hyaluronic acid dissolved to be absorbed into the living body. In contrast, with respect to a dog whose chest was closed without placing the anti-adhesion film, it was observed that adhesion had occurred between the portion sutured after the thoracotomy and the surface of the lung.

### Industrial Applicability

The medical/cosmetic material according to the present invention is useful as a material for constituting an anti-adhesion material.

## Claims

1. A medical/cosmetic material comprising:
a base comprising a water-soluble salt of a first polyanionic polysaccharide; and
a powder dispersed in the base and formed by water-insolubilizing a powder of a water-soluble salt of a second polyanionic polysaccharide with a treatment liquid comprising an acid anhydride.

2. The medical/cosmetic material according to claim 1, wherein the base is an aqueous solution of the water-soluble salt of the first polyanionic polysaccharide.

3. The medical/cosmetic material according to claim 1, wherein the base is a film-like shaped body made of a raw material comprising the water-soluble salt of the first polyanionic polysaccharide.

4. The medical/cosmetic material according to any one of claims 1 to 3, wherein the first polyanionic polysaccharide and the second polyanionic polysaccharide are each at least one selected from the group consisting of hyaluronic acid, carboxymethyl cellulose, and alginic acid.

5. The medical/cosmetic material according to any one of claims 1 to 4, wherein the acid anhydride is at least any one of acetic anhydride and propionic anhydride.

6. An anti-adhesion material comprising a polyhydric alcohol or a polyhydric alcohol aqueous solution retained in the medical/cosmetic material according to any one of claims 3 to 5.
